# EUROPEAN PATENT APPLICATION

(11) **EP 1 676 575 A2**
(43) Date of publication of application: **05.07.2006**
(21) Application number: 04792783.5
(22) Date of filing: 21.10.2004
(51) Int. Cl.: A61K 31/4196, A61P 43/00, A61P 11/00, A61P 11/10, A61P 29/00, A61P 31/06, C07D 403/04

(54) **MUCIN PRODUCTION INHIBITOR**

(30) Priority: 22.10.2003 JP 2003362538
(71) Applicant: TAIHO PHARMACEUTICAL COMPANY LIMITED, Chiyoda-ku, Tokyo 101-8444 (JP)
(72) Inventor: SASAKI, Eiji, C/O TAIHOPHARMACEUTICAL CO., LTD., Tokyo 101-0054 (JP); KINIWA, Mamoru, C/O TAIHOPHARMACEUTICAL CO., LTD., Hanno-shi, Saitama 357-0041 (JP)
(74) Representative: Blodig, Wolfgang
(86) International application number: PCT/JP2004/015634
(87) International publication number: WO 2005/041858

(57) **Abstract**

To provide a drug useful for prevention or treatment of respiratory diseases accompanying hyper-secretion of mucus, the drug normalizing physiological functions of the respiratory tract by preventing over-production of mucin from the respiratory tract epithelium. A mucin production inhibitor comprising, as an active ingredient, a benzimidazole derivative represented by formula (1) : 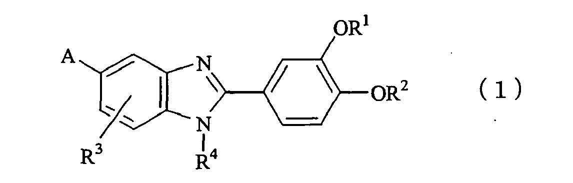 (wherein A represents a triazole group; R¹ and R² may be identical to or different from each other, and each represents an aliphatic hydrocarbon group which may have an alicyclic hydrocarbon group, or an alicyclic hydrocarbon group; R³ represents a hydrogen atom or a substituent; and R⁴ represents a hydrogen atom or a protective group for the nitrogen atom) or a pharmacologically acceptable salt thereof.

## Description

### Technical Field

The present invention relates to a mucin production inhibitor which inhibits production of mucin in the respiratory tract epithelium and which is useful for prevention or treatment of respiratory diseases accompanying hyper-secretion of mucus.

### Background Art

The primary function of the lungs is to smoothly conduct ventilation and gas exchange so as to ensure normal supply of oxygen and removal of carbon dioxide within the tissues of the body, to thereby produce and maintain energy of the living body. To this end, inhalation of outside air into the pulmonary alveolus area that serves as the gas exchange unit must be performed most efficiently. Accordingly, resistance to air flow can be minimized only when a sufficient capacity is secured for the inner cavity. Therefore, it is necessary to adequately control respiratory tract secretion over the entire length between the central portion of the respiratory tract and the peripheries of the respiratory tract. Airway secretions play important roles in functioning as a physical barrier to protect the living body by covering the respiratory tract tissue, transporting and eliminating, by way of mucociliary transportation, foreign substances in cooperation with ciliary movements of the respiratory tract epithelial cells, and maintaining topical homeostasis. However, when the amount of respiratory tract secretions is excessive, sputa accumulated within the respiratory tract become hotbeds for bacterial proliferation, leading to repeated respiratory tract infections. In addition, decrease in the cross-sectional area of the respiratory tract cavity results in aggravation of obstructive ventilation disorders or the like, inviting aggravation of chronic respiratory tract diseases. Specifically, such narrowing is known to be closely related to aggravation in the pathological condition of respiratory diseases such as chronic obstructive pulmonary disease, chronic respiratory tract infection, chronic bronchitis, chronic sinusitis, pneumonia, pulmonary tuberculosis, pneumoconiosis, and pulmonary fibrosis (Jun Tamaoki, *Saishin Iyaku,* 2002, vol. 57 No. 10, pp. 99-105; Hirotoshi Matsui, Medicine and Drug Journal, 2002, vol. 38 No. 12, pp. 133-138; or Yuichi Majima, *Arerugi To Rinsho,* 2002, vol. 22 No. 2, pp. 16-21).

Among the above-mentioned diseases, COPD (chronic obstructive pulmonary disease) is a disease characterized by "not completely reversible air flow limitation which is typically progressive and is associated with abnormal inflammatory responses to hazardous particles or gases." In a lung function test, a COPD patient shows an obstructive ventilation disorder; in other words, every time when the patient exhales forcibly after having breathed in deeply, he will have a reduced volume of exhaled air during the initial one second.

This disease has a poor prognosis, and responses to drugs are limited. Currently, COPD is the fourth leading cause of death in the world, and the morbidity rate and death rate are expected to increase further. Among several factors that aggravate the pathological profiles of COPD is hyper-secretion of mucos from the epithelium of the respiratory tract. That is, impairment of mucociliary clearance causes accumulation and difficult expectoration of overly-produced sputum, inducing further obstructive ventilation disorders and aggravation of symptoms. Therefore, control of such a state of overproduction of mucus is a critical strategy for achieving successful treatment of COPD (Jun Tamaoki, *Saishin Iyaku,* 2002, vol. 57 No. 10, pp. 99-105). Anti-cholinergic drugs have been used for treating COPD, but these are employed for the purpose of relieving the conditions on the basis of their bronchodilatative action. Thus, they constitute only part of symptomatic therapy, and do not arrest progress or aggravation of the pathological conditions. Therefore, also from the viewpoint of long-term prognosis with satisfactory QOL, there is keen demand for COPD therapeutic drugs which functions to control overproduction of mucus.

Diseases which cause chronic airway infections include cystic pulmonary fibrosis, diffuse panbronchiolitis, primary ciliary dyskinesia, bronchiectasis, chronic bronchitis, chronic sinusitis, and the like. Researchers consider that, when coupled with bacterial infection, these diseases develop hyperactivity of the mucus production system in the respiratory tract and impairment of mucociliary clearance, which in turn form a vicious circle of inducing further disorder of the respiratory tract epithelium (Hirotoshi Matsui, Medicine and Drug Journal, 2002, vol. 38 No. 12, pp. 133-138) .

Chronic bronchitis is defined to be a pathological condition which involved "persistent expectoration of sputum over 2 years or longer periods." Thus, chronic bronchitis is a disease characterized mainly by long-term hyperfunction of airway secretion. In chronic bronchitis, airway secretions generally have high viscosity, and therefore, in association with lowered ciliary motility caused by injury of the mucous membrane of the respiratory tract, give rise to disturbance in expectoration of sputum. Also, hindered mucociliary transport contributes repetition of respiratory tract infections, and when overly produced secretions are accumulated in the bronchial lumens, obstructive ventilation disorders are induced, to possibly cause acute aggravation of the disease.

Chronic sinusitis represents chronic inflammation occurred in the nasal sinus, which is present in the inner deep site of the nose. Generally, sinusitis passes into a chronic state, beginning with acute inflammation such as common cold followed by repeated bacterial infections attributed to influenza viruses or other microorganisms. As a result, pus or mucus cannot be discharged, permitting accumulation thereof in the nasal sinus to eventually develop the symptoms. If sinusitis continues for a long time, nasal discharge tends to run down from the deep inner site of the nose to the throat, and the amount of sputum increases, to possibly cause chronic pharyngolarynitis and chronic bronchitis.

Other respiratory diseases exhibiting hyper-secretion of mucus include, but not limited to, pneumonia, pulmonary tuberculosis, pneumoconiosis, and pulmonary fibrosis.

Patients suffering any of the above-mentioned respiratory diseases accompanying overproduction of mucus, such as COPD, chronic bronchitis, diffuse panbronchiolitis, cystic pulmonary fibrosis, or chronic sinusitis present, as histological features of the respiratory tract, Brunner's gland hypertrophy and metaplasia of goblet cells. The mucus is primarily constituted by aqueous matter containing electrolytes and mucin, which is a glycoprotein. Overproduction of mucin and change in physical characteristics (fluidity, viscosity, and spinnability) of mucin are considered to be closely related to the onset of a variety of respiratory diseases, persistence in the chronic state, and formation of the intractable state. That is, overproduction of mucin impairs the physiological mucociliary clearance, causing clogging of mucus, entrapment of air, and eventually atelectasis which accompanies infection, thereby leading to unfavorable prognosis (see, for example, Vestbo J. et al., Am J Respir Crit Care Med., 1996, vol. 153 No. 5, pp. 1530-1535). Accordingly, it is considered that inhibition of metaplasia of goblet cells, which participate in production and secretion of mucin, a primary component of the mucus, or inhibition of biosynthesis of mucin is believed to normalize physiological functions of the respiratory tract caused by such overproduction or hyper-secretion of mucus, to thereby contribute to prevention and treatment of diseases associated therewith.

Expectorants and mucolytic agents have been used in clinical settings to treat hyper-secretion of mucus in the respiratory tract epithelium. These drugs act to promote discharge of mucous secretions from the respiratory tract, or decrease viscosity or elasticity thereof. However, these drugs do not inhibit the synthesis of mucin or overproduction of mucus. Presently, among drugs that directly act to inhibit secretion of mucin, only steroids have clinical indications (e.g., Hiroko Nogami, et al., Medicine and Drug Journal, 2002, vol. 38 No. 12, pp. 127-132). However, steroids, which are immunosuppressants, have drawbacks including bone loss and retarded growth. Also, the primary action of macrolide antibiotics, which are often used to treat bacterium-caused hyper-secretion of mucus, is to mitigation of symptoms through sterilization. Accordingly, there exists strong demand to develop preventive and therapeutic drugs for respiratory diseases which can inhibit overproduction of mucin with minimal side effects.

Meanwhile, a benzimidazole derivative serving as an active ingredient of the drug of the present invention is known as an anti-allergic agent or an anti-inflammatory agent having a phosphodiesterase IV (PDE (IV)) inhibitory effect and an interleukin (IL)-4 production inhibitory effect (Japanese Patent No. 3271991). However, that the derivative has a direct effect of inhibiting mucin production in the epithelium of respiratory tract has remained unknown. Generally, it has been known that increase in the amount of intracellular cyclic AMP (cAMP) results in increased secretion of mucin. Since PDE (IV) is one of degradation enzymes acting on intracellular cAMP, inhibition of PDE (IV) is considered to serve as a driving force for increasing secretion of mucin (Duncan F. Rogers, Current Allergy and Asthma Reports, 2002, vol. 3, pp. 238-248). Therefore, no one would predict that the above-mentioned benzimidazole derivative having the PDE (IV) inhibitory effect would have a direct inhibitory action against mucin production. In addition, since IL-4 participates in the formation of pathologies of allergic diseases, IL-4 production inhibitory action is considered to contribute to treatment of allergic diseases. However, regarding the aforementioned non-allergic diseases, no one has succeeded in estimating obtainable therapeutic effect which is derived from mucin production inhibition.

### Disclosure of the Invention

An object of the present invention is to provide a drug useful for prevention or treatment of respiratory diseases accompanying hyper-secretion of mucus, the drug normalizing physiological functions of the respiratory tract by preventing overproduction of mucin from the respiratory tract epithelium.

The present inventors have conducted extensive studies with an aim to attain the above object, and have found that the benzimidazole derivatives represented by formula (1) exhibit an excellent mucin production inhibitory effect and are useful for prevention or treatment of respiratory diseases accompanying hyper-secretion of mucus.

Accordingly, the present invention provides a mucin production inhibitor comprising, as an active ingredient, a benzimidazole derivative represented by formula (1): 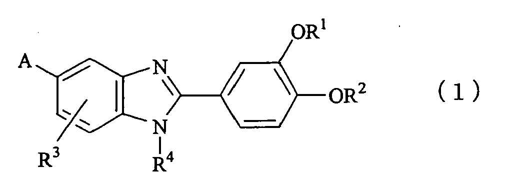 (wherein A represents a triazole group; R¹ and R² may be identical to or different from each other, and each represents an aliphatic hydrocarbon group which may have an alicyclic hydrocarbon group, or an alicyclic hydrocarbon group; R³ represents a hydrogen atom or a substituent; and R⁴ represents a hydrogen atom or a protective group for the nitrogen atom) or a pharmacologically acceptable salt thereof.

The present invention also provides an agent for ameliorating symptoms in relation to a respiratory disease accompanying hyper-secretion of mucus from the respiratory tract epithelium: stagnation of secreted respiratory tract mucus; difficulty in sputum expectoration; or accumulation of sputum, the agent comprising, as an active ingredient, a benzimidazole derivative represented by formula (1) or a pharmacologically acceptable salt thereof.

The present invention also provides an expectorant comprising, as an active ingredient, a benzimidazole derivative represented by formula (1) or a pharmacologically acceptable salt thereof.

The present invention also provides a method for treating a respiratory disease accompanying hyper-secretion of mucus from the respiratory tract epithelium,
the method comprising administering, in an effective amount, a benzimidazole derivative represented by formula (1) or a pharmacologically acceptable salt thereof.

The present invention also provides a method for ameliorating a symptom in relation to a respiratory disease accompanying hyper-secretion of mucus from the respiratory tract epithelium, the symptom being selected from among stagnation of secreted respiratory tract mucus, difficulty in sputum expectoration, and accumulation of sputum,
the method comprising administering, in an effective amount, a benzimidazole derivative represented by formula (1) or a pharmacologically acceptable salt thereof.

The present invention also provides use of a benzimidazole derivative represented by formula (1) or a pharmacologically acceptable salt thereof for the manufacture of mucin production inhibitor.

The present invention further provides use of a benzimidazole derivative represented by formula (1) or a pharmacologically acceptable salt thereof for the manufacture of an agent for ameliorating a symptom in relation to a respiratory disease accompanying hyper-secretion of mucus from the respiratory tract epithelium, the symptom being selected from among stagnation of secreted respiratory tract mucus, difficulty in sputum expectoration, and accumulation of sputum.

According to the present invention, production of mucin from the respiratory tract epithelium can be suppressed. Thus, the invention is useful for preventing or treating respiratory diseases accompanying hyper-secretion of mucus; such as COPD, chronic respiratory tract infections, chronic bronchitis, chronic sinusitis, pneumonia, pulmonary tuberculosis, pneumoconiosis, and pulmonary fibrosis. Particularly, the present invention can provides amelioration of symptoms in relation to the respiratory disease such as stagnation of secreted respiratory tract mucus; difficulty in sputum expectoration; and accumulation of sputum, and provide a drug effective as an expectorant. Thus, the invention contributes to the improvement of long-term prognostic conditions with satisfactory QOL.

### Brief Description of the Drawings

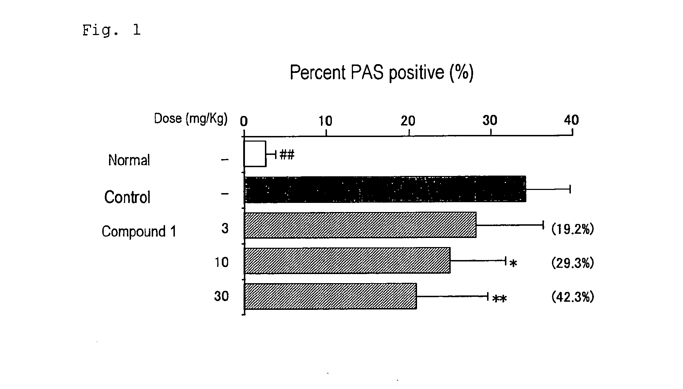

Fig. 1 is a graph showing an inhibitory effect of compound 1 on hyperplasia of goblet cells.
*: P<0.05, **: P<0.01, comparison with control (Dunnett test),
##: P<0.01, comparison with control (Welch test), the value in each parenthesis represents percent inhibition (%).

### Best Modes for Carrying Out the Invention

The benzimidazole derivative of the present invention represented by formula (1) or a pharmacologically acceptable salt thereof exhibits an excellent mucin production inhibitory effect in respiratory tract epithelial cells, as described in the Examples.

When mucin is over-produced through hyperplasia of goblet cells, physiological mucus-ciliary clearance is inhibited, resulting in atelectasis involving stagnation of mucus, capture of air, and an infection coincident therewith. Therefore, use of the benzimidazole derivative exerting the aforementioned action can activate mucus-ciliary clearance and improve functions of the respiratory tract, whereby respiratory diseases accompanying hyper-secretion of mucus can be prevented or treated.

Examples of the respiratory diseases accompanying hyper-secretion of mucus include chronic obstructive pulmonary disease (COPD), chronic respiratory tract infections, chronic bronchitis, chronic sinusitis, pneumonia, pulmonary tuberculosis, pneumoconiosis, and pulmonary fibrosis. Examples of the respiratory diseases preferred in the present invention include chronic obstructive pulmonary disease, chronic respiratory tract infections, and chronic bronchitis. The respiratory diseases further includes cystic pulmonary fibrosis (CF), diffuse panbronchiolitis (DPB), primary ciliary dyskinesia (PCD), bronchiectasis, chronic bronchitis, and chronic sinusitis, which cause a chronic respiratory tract infection.

The benzimidazole derivative of the present invention represented by formula (1) or a pharmacologically acceptable salt thereof is effective for preventing or treating the aforementioned respiratory diseases, and particularly effective for ameliorating symptoms in relation to the above respiratory diseases; e.g., stagnation of secreted respiratory tract mucus, difficulty in sputum expectoration, and accumulation of sputum. Thus, the derivative or a salt thereof is also useful as an expectorant.

In addition, the benzimidazole derivative of the present invention represented by formula (1) or a pharmacologically acceptable salt thereof is also useful as an agent for preventing or treating exudative otitis media, which is caused by accumulation of mucus occurring after bacterial infection.

In the benzimidazole derivatives of the present invention represented by formula (1), examples of the triazole group of A include 1,2,4-triazol-1-yl, 1,2,4-triazol-4-yl, 1,2,3-triazol-1-yl, and 1,2,3-triazol-2-yl. Of these, 1,2,4-triazol-1-yl and 1,2,4-triazol-4-yl are preferred, with 1,2,4-triazol-1-yl being more preferred.

Examples of the aliphatic hydrocarbon group in R¹ or R² include linear- or branched-chain lower alkyl groups having 1 to 6 carbon atoms, such as methyl, ethyl, n-propyl, isopropyl, n-butyl, isobutyl, sec-butyl, tert-butyl, n-pentyl, isopentyl, 3-pentyl, and n-hexyl; and linear- or branched-chain lower alkenyl groups having 2 to 6 carbon atoms, such as vinyl, 1-propenyl, allyl, dimethylallyl, isopropenyl, 1-butenyl, 2-butenyl, 1-methyl-2-butenyl, 1,3-butanedienyl, 1-pentenyl, 2-pentenyl, 2-hexenyl, and 1,4-hexanedienyl. Of these, linear- or branched-chain lower alkyl groups having 1 to 6 carbon atoms are preferred, with methyl, isopropyl, and 3-pentyl being more preferred.

Examples of the aliphatic hydrocarbon group having an alicyclic hydrocarbon group according to R¹ or R² include the aforementioned aliphatic hydrocarbon groups further having monocyclic alicyclic hydrocarbon groups having 3 to 7 carbon atoms, which may have a linear- or branched-chain saturated lower alkyl group having 1 to 3 carbon atoms, such as cyclopropyl, cyclobutyl, cyclopentyl, cyclopentenyl, cyclohexyl, 3-isopropyl-cyclohexyl, cyclohexenyl, 2-methyl-2-cyclohexenyl, 3-methyl-2-cyclohexenyl, 4-ethyl-2-cyclohexenyl, cycloheptanyl, and cycloheptenyl; or having alicyclic hydrocarbon groups of cross-linked ring or polycyclic system, such as bicyclobutanyl, bicyclooctanyl, norbornyl, norborenyl, and indanyl. Of these, linear- or branched lower alkyl groups having 1 to 6 carbon atoms substituted with a monocyclic alicyclic hydrocarbon group having 3 to 7 carbon atoms are preferred, with cyclopropylmethyl and cyclopentylmethyl being more preferred.

Examples of the alicyclic hydrocarbon group according to R¹ or R² include the aforementioned monocyclic alicyclic hydrocarbon groups having 3 to 7 carbon atoms, which may have a linear- or branched-chain lower alkyl group having 1 to 3 carbon atoms, and the aforementioned alicyclic hydrocarbon groups of cross-linked ring or polycyclic system. Of these, monocyclic alicyclic hydrocarbon groups having 3 to 7 carbon atoms are preferred, with cyclopentyl, cyclopentenyl, cyclohexenyl, and cycloheptenyl being more preferred.

R³ is a hydrogen atom or a substituent, and examples of the substituent include a lower alkoxy group, a lower alkyl group, a hydroxy group, a nitro group, a cyano group, an amino group, and a halogen atom, but R³ is preferably a hydrogen atom or a lower alkoxy group. Examples of the lower alkoxy group include linear- or branched-chain alkoxy groups having 1 to 6 carbon atoms, such as methoxy, ethoxy, n-propoxy, isopropoxy, n-butoxy, isobutoxy, sec-butoxy, tert-butoxy, n-pentyloxy, cyclopentyloxy, isopentyloxy, n-hexyloxy, and cyclohexyloxy. Of these, methoxy, ethoxy, and pentyloxy are preferred, with methoxy and n-pentyloxy being more preferred. Examples of the lower alkyl group include linear- or branched-chain alkyl groups having 1 to 6 carbon atoms, such as methyl, ethyl, n-propyl, isopropyl, n-butyl, isobutyl, sec-butyl, tert-butyl, n-pentyl, isopentyl, and n-hexyl. Of these, methyl and ethyl are preferred. Examples of the halogen atom include fluorine, chlorine, bromine, and iodine.

The substituent of R³ can be substituted at least at one of the 4-, 6-, and 7-positions of the benzimidazole nucleus, but preferably at the 6-position.

The nitrogen atom-protective group of R⁴ may be any group which can be hydrolyzed easily in the living body, and its examples include acyl groups such as acetyl, benzoyl, and pivaloyl; alkoxycarbonyl groups such as methoxycarbonyl, ethoxycarbonyl, and benzyloxycarbonyl; aryloxycarbonyl groups such as phenoxycarbonyl; linear- or branched-chain lower alkyl groups having 1 to 6 carbon atoms, such as methyl, ethyl, n-propyl, isopropyl, n-butyl, isobutyl, sec-butyl, tert-butyl, n-pentyl, isopentyl, and n-hexyl; hydroxy lower alkyl groups such as hydroxymethyl and hydroxyethyl; aralkyl groups such as benzyl and trityl; alkoxyalkyl groups such as methoxymethyl, ethoxymethyl, methoxyethyl, and ethoxyethyl; alkoxyalkoxyalkyl groups such as methoxymethoxymethyl, methoxyethoxymethyl, ethoxyethoxymethyl, and ethoxyethoxyethyl; and aralkyloxyalkyl groups such as benzyloxymethyl, trityloxymethyl, berizyloxyethyl, and trityloxyethyl.

Among the aforementioned benzimidazole derivatives, compounds having asymmetric carbons exist in optical isomer forms and geometrical isomer forms depending on the number of asymmetric carbons, and all of these isomers are included in the present invention.

Though conventionally used optical resolution methods can be used for the isolation of optically active substances, the optically active substances can also be obtained by fractionating them by means of HPLC employing an optically active column. An example of the optically active column is CHIRALPAK AD manufactured by Daicel Chemical Industries.

Examples of the salt of the benzimidazole derivative (1) of the present invention include pharmacologically acceptable salts. Specific examples include mineral acid salts such as hydrochloride, sulfate, and nitrate; and organic acid salts such as fumarate, maleate, tartarate, toluenesulfonate, and methanesulfonate.

The benzimidazole derivative of the present invention may exist in tautomer forms based on the benzimidazole skeleton, and such isomers are also included in the present invention. Also, the compound of the present invention includes solvates thereof such as hydrates and amorphous or polymorphic forms thereof.

In the aforementioned formula (1), preferred is a combination where A is 1, 2, 4-triazol-1-yl, R¹ and R² are identical to or different from each other, and each represents a linear or branched lower C1-C6 alkyl group which may have a C3-C7 mono-ring alicyclic hydrocarbon group, or a C3-C7 mono-ring alicyclic hydrocarbon group, preferably, methyl, isopropyl, 3-pentyl, isopentyl, cyclopropylmethyl, cyclopentylmethyl, cyclopentyl, cyclopentenyl, cyclohexenyl, and cycloheptenyl, R³ is a hydrogen atom, and R⁴ is a hydrogen atom. Particularly preferred compounds are the following compounds 1 to 6 or a salt thereof.

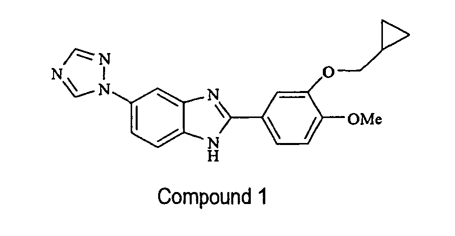

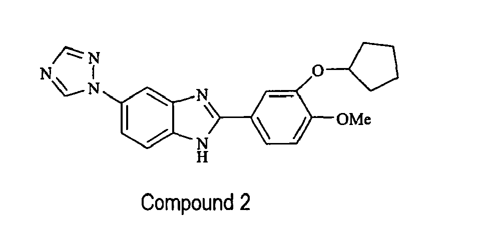

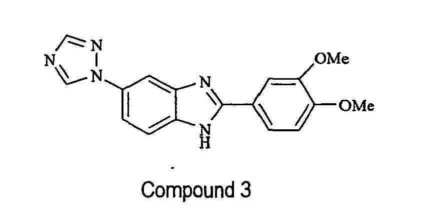

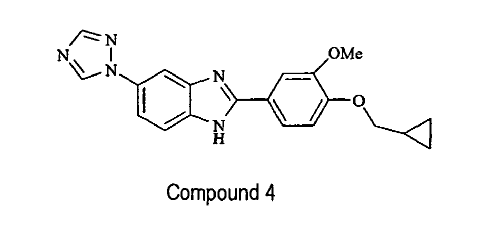

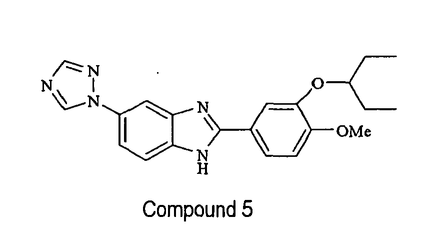

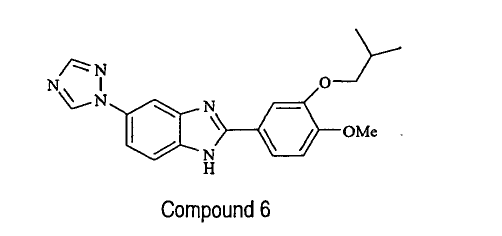

The benzimidazole derivatives represented by the above formula (1) are a known compound. Thus, any of the benzimidazole derivatives can be produced through known methods (see, Japanese Patent No. 3271991).

The mucin production inhibitor, the drug for improving a symptom or the expectorant of the present invention can be made into pharmaceutical preparation compositions through a routine method employing appropriate pharmaceutical carriers. Examples of the carriers to be employed include those which are generally used in drugs, such as a filler, a binder, a disintegrating agent, a lubricant, a coloring agent, a flavor corrective, an order corrective and a surfactant.

Dosage form of the mucin production inhibitor, the agent for improving a symptom, or the expectorant of the present invention when used as a therapeutic agent in mammals including human is not particularly limited and can be selected optionally depending on each therapeutic purpose. Examples include parenteral preparations such as injections, suppositories, and external preparations (e.g., ointments and adhesives); and oral preparations such as tablets, coated tablets, powders, granules, capsules, solutions, pills, suspensions, and emulsions. The dosage form may be an inhalation preparation. Examples of preferred inhalation preparations include aerosol preparations, powder inhalation preparations, and liquid inhalation preparation.

The aforementioned various drugs may be made into pharmaceutical preparations by ordinary preparation methods known in the field.

In forming oral solid dosage forms such as tablets, powders, and granules, examples of the carriers to be employed include fillers such as lactose, sucrose, sodium chloride, glucose, urea, starch, calcium carbonate, kaolin, crystalline cellulose, silicic acid, methyl cellulose, glycerol, sodium alginate, and acacia; binders such as simple syrup, glucose solution, starch solution, gelatin solution, polyvinyl alcohol, polyvinyl ether, polyvinyl pyrrolidone, carboxymethyl cellulose, shellac, methyl cellulose, ethyl cellulose, hydroxypropyl cellulose, water, ethanol, and potassium phosphate; disintegrating agents such as dry starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch, and lactose; disintegration inhibitors such as sucrose, stearic acid, cacao butter, and hydrogenated oil; absorption accelerating agents such as quaternary ammonium base and sodium lauryl sulfate; moisture keeping agents such as glycerol and starch; adsorbents such as starch, lactose, kaolin, bentonite, and colloidal silica; and lubricants such as purified talc, stearic acid salt, boric acid powder, and polyethylene glycol.

As occasion demands, tablets can be made into coated tablets using usual coatings, such as sugar coated tablets, gelatin coated tablets, enteric coated tablets, film coated tablets, double-layer tablets, and multi-layer tablets. In forming the dosage form of pills, examples of the carriers to be employed include fillers such as glucose, lactose, starch, cacao butter, hardened plant oil, kaolin, and talc; binders such as powdered acacia, powdered tragacanth, gelatin, and ethanol; and disintegrating agents such as laminaran and agar.

Capsules are prepared by mixing the active ingredient with the aforementioned various carriers and filling appropriate capsules such as hard gelatin capsules or soft capsules with the mixture.

The dosage form of suppositories can be formed by adding an appropriate absorption accelerating agent to carriers such as polyethylene glycol, cacao butter, lanolin, higher alcohol, higher alcohol esters, gelatin, semisynthetic glyceride, and Witepsol (registered trademark of Dynamit Nobel A.G.).

Examples of the carriers to be used in forming the dosage form of injections include diluents such as water, ethyl alcohol, macrogol, propylene glycol, ethoxylated isostearyl alcohol, polyoxylated isostearyl alcohol, and polyoxyethylene sorbitan fatty acid esters, pH adjusting agents and buffers such as sodium citrate, sodium acetate, and sodium phosphate, and stabilizing agents such as sodium pyrosulfite, ethylenediaminetetraacetic acid, thioglycollic acid, and thiolactic acid. In this case, the pharmaceutical preparation may contain sodium chloride, glucose or glycerol in an amount sufficient for preparing isotonic solution. It may also contain other additives such as a solubilization assisting agent, a soothing agent, and a local anesthetic. By adding these carriers, subcutaneous, intramuscular, and intravenous injections can be produced through a routine method.

The liquid preparations may be aqueous or oily suspensions, solutions, syrups or elixirs, and they are prepared using general additive agents through a routine method.

When the dosage form of ointments such as pastes, creams, and gels are prepared, generally employable materials such as a base, a stabilizing agent, a moistening agent, and a preservative are formulated and mixed in accordance with need and made into respective preparations. Examples of the base to be employed include white petrolatum, paraffin, glycerol, a cellulose derivative, polyethylene glycol, silicon, and bentonite. Examples of the preservative to be employed include methyl parahydroxybenzoate, ethyl parahydroxybenzoate, and propyl parahydroxybenzoate.

Adhesive preparations may be produced through a routine method by coating the aforementioned ointments such as creams, gels, or pastes on a conventional support. Examples of the suitable support include woven or non-woven fabrics made of cotton, staple fiber, or chemical fiber and films and foam sheets of polymers such as soft vinyl chloride, polyethylene, and polyurethane.

In order to produce an inhalation preparation, the benzimidazole derivative (1) or a pharmacologically acceptable salt thereof is added to purified water or distilled water for injection, and the mixture is stirred for dissolution. If desired, the preparation may further contain a generally employable additive such as an osmotic agent (e.g., sodium chloride), a buffer (e.g., boric acid, phosphoric acid-sodium hydrogenphosphate, or sodium dihydrogenphosphate), preservative (e.g., benzalkonium chloride), or a thickener (e.g., carboxy-vinyl polymer). A liquid inhalation preparation is administered by means of an inhalation apparatus such as Nubulizer (registered trademark). In order to prepare an aerosol, the benzimidazole derivative (1) of the present invention or a pharmacologically acceptable salt thereof is micro-pulverized preferably to a micropowder of 5 µm or less, and the micropowder and an optional dispersant are charged into a spraying apparatus together with a spray medium under cooling. Alternatively, the benzimidazole derivative (1) of the present invention or a pharmacologically acceptable salt thereof is dissolved in an organic solvent (e.g., ethanol)-water mixture, and the solvent is removed under heated and reduced pressure conditions. The thus-produced compound may also be micro-pulverized to produce a micropowder to be charged to a spraying apparatus. Examples of the spray medium preferably employed in the aerosol preparation include liquefied hydrofluoroalkanes; e.g., HFA134a (1, 1, 1, 2-tetrafluoroethane (CH₂FCF₃) , and HFA227 (1, 1, 1, 2, 3, 3, 3-heptafluoropropane (CF₃-CHF-CF₃)). These liquefied hydrofluoroalkanes may be used singly or in combination of two or more species. Examples of preferred dispersants include middle-chain fatty acid triglycerides (e.g., Miglyol 812 (trademark of Dynamit Nobel A.G.) and soybean lecithin. A powder inhalation preparation is produced from miropowder of the benzimidazole derivative (1) of the present invention or a pharmacologically acceptable salt thereof, the micropowder produced in a manner similar to that employed in the case of the aerosol preparation and by mixing with an optional excipient such as lactose, if needed. The powder inhalation preparation is administered by use of an inhalation apparatus such as Spinhaler (registered trademark).

The amount of the benzimidazole derivative (1) of the present invention or a salt thereof to be contained in the aforementioned pharmaceutical preparations varies depending on various conditions such as dosage form, route of administration and dosage regimen, so that the amount cannot be defined in a wholesale manner and should be selected from a broad range for each case, but the pharmaceutical preparations may contain the derivative generally in an amount of approximately from 1 to 70% by weight.

Administration methods of the aforementioned pharmaceutical preparations, such as intestinal application, oral administration, rectal administration, buccal application and percutaneous absorption, are not particularly limited but optionally decided depending, for example, on the dosage form, the age, sex, and other conditions of each patient and the degree of symptoms of each patient. For example, in the case of tablets, pills, solutions, suspensions, emulsions, granules, and capsules, they are orally administered, and suppositories are used by rectal administration. In the case of injections, they are administered by intravenous injection as such or after mixing with a usual replacement solution such as of glucose or amino acids or, as occasion demands, administered alone by intraarterial infusion, intramuscular injection, intracutaneous injection, subcutaneous injection, or intraperitoneal injection. Ointments are applied, for example, to the skin or oral mucous membrane. The inhalation preparation may be administered through, for example, intranasal inhalation or via the upper respiratory tract and/or the lower respiratory tract.

Dose of the active ingredient of the mucin production inhibitor of the present invention is optionally selected based on the application method, the age, sex, morbid state of each patient, kind of the benzimidazole derivative of the present invention or a salt thereof to be administered and other conditions, but it may be within a range of generally from about 0.1 to 1,000 mg/kg/day, preferably from about 0.5 to 500 mg/kg/day. These pharmaceutical preparations of the present invention can be administered once per day by dividing the daily dose into 2 to 4 doses per day.

### Examples

The present invention will next be described in more detail by way of Test Examples and Drug Preparation Examples. Test Example 1

### Mucin production inhibitory action

The test was carried out in accordance with a partially modified method of Takeyama *et al.* (Proc. Natl. Acad. Sci. USA, Vol. 96, P. 3081-3086, 1999). The specific procedure will be described as follows. NCI-H292 cells, which are human respiratory tract epithelial cell lines, were suspended in a 10%FCS-containing RPMI 1640 culture medium, and disseminated to a 12-well culture plate so that the cell concentration of each well was adjusted to 2 x 10⁵ cell/well, followed by culturing for two days. Subsequently, the culture medium was changed to a 0.2%FCS-containing RPMI culture medium, followed by culturing for another two days. The thus-cultured cells were employed in the test. Each test drug and EGF (25 ng/mL) were added to each well, and the mucin level of the supernatant of the culture 48 hours after initiation of culturing was determined through the ELISA method. Percent inhibition (%) with regard to control value was calculated by supposing the difference produced by subtracting normal value from control value to be 100%. In this case, the control value is defined as the mucin amount in the culture supernatant to which only EGF was added, while the normal value is defined as the muchin amount in the culture supernatant containing neither of test drug and EGF. In addition, cilomilast (J. Pharmacol. Exp. Ther., Vol. 284, p. 420-426, 1998) and roflumilast (J. Pharmacol. Exp. Ther., Vol. 297, p. 267-279, 2001), which are typical phosphodiesterase (IV) inhibitors were also tested in a similar manner. Table 1 shows the results.

As is clear from the results, the compound of the present invention inhibited production of mucin in a concentration-dependent manner, while cilomilast and roflumilast, having phosphodiesterase (IV) inhibitory action, promoted production of mucin, in contrast to the case of the compound of the present invention.

**Table 1**

| Compound No. | Structure | Concentration(µM) | Percent inhibition of mucin production (%) |
|---|---|---|---|
| 1 | 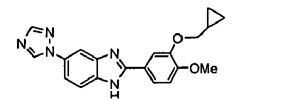 | 1 | 18.1 |
| | | 3 | 49.4 |
| | | 10 | 88.6 |
| 2 | 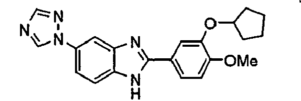 | 1 | 57.1 |
| | | 3 | 53.3 |
| | | 10 | 80.8 |
| 3 | 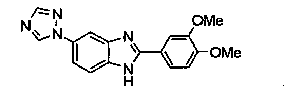 | 1 | 60.0 |
| | | 3 | 74.9 |
| | | 10 | 83.9 |
| 4 | 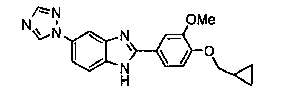 | 1 | 22.7 |
| | | 3 | 48.8 |
| | | 10 | - |
| 5 | 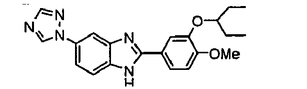 | 1 | 15.4 |
| | | 3 | 40.2 |
| | | 10 | 65.9 |
| 6 | 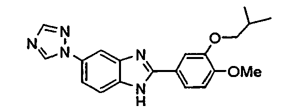 | 1 | 15.4 |
| | | 3 | 51.4 |
| | | 10 | 102.2 |
| Cilomilast | 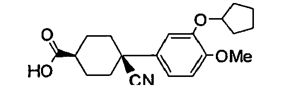 | 1 | -21.4 |
| | | 3 | -17.2 |
| | | 10 | -35.2 |
| Roflumilast | 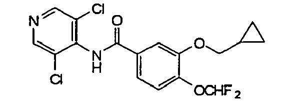 | 1 | -37.4 |
| | | 3 | -28.7 |
| | | 10 | -16.8 |

### Test Example 2

Inhibitory effect on hyperplasia of goblet cells in the respiratory tract epithelium.

The test was carried out in accordance with a partially modified method of Matsubara *et al*. (Int. Arch. Allergy Immunol., Vol. 116, p. 67-75, 1998). The specific procedure will be described as follows. To each of the 7-week old male Lewis Wistar rats, a Sephadex suspension (0.1 mg/mL/head) was intravenously administered (total three times: day 0, 2, and 5). Two days after the final administration, the rat was slaughtered by exsanguination under anesthetic with pentobarbital. The lung tissue was extirpated from the rat and immersed in a neutral formalin buffer for fixation. The superior lobe of the left lung was cut to prepare a sliced sample, which was then stained with periodic acid-Schiff (PAS). The ratio of the PAS-stained positive area to the respiratory tract epithelium was calculated by use of an image analysis software (Winroof). Each test compound was suspended in 0.5% hydroxypropylmethyl cellulose (HPMC), and the suspension was orally administered once per day for continuous seven days from the start of administration of Sephadex. The results are shown in Fig. 1. In the Figure, numerical data are represented by mean value ± standard deviation (each group: 11-12 samples).

### Test Example 3

### Inhibitory effect on hyper-secretion of respiratory tract mucus

The procedure of the test was based on a partially modified method of Tesfaigzi *et al*. (Am. J. Physiol. Lung Cell Mol. Physiol., Vol. 279, p. L1210-L1217, 2000). The specific procedure will be described as follows. To each of the 7-week male Brown Norway rats, LPS (derived from *E*. *coli.,* strain 0111: B4) 100 µg was intratracheally administered under anesthetic with ether, thereby inducing inflammation. Two days after administration of LPS, the rat was slaughtered under anesthetic with pentobarbital, and the extirpated respiratory tract was washed with PBS containing 5mM EDTA and 5mM dithiothreitol (3 mL). The amount of mucus contained in the collected BALF was determined through a lectin assay method employing UEA-1. Each test drug was suspended in a 0.5% HPMC, and the suspension was orally administered one hour before administration of LPS and on the following day. The results are shown in Table 2. In Table 2, numerical data are represented by mean value ± standard deviation (each group: 6 samples). Percent inhibition (%) was calculated in the same way as in Test Example 1.

**Table 2**

| Group | Mucus level of BALF (ng/mL) | Percent inhibition (% ) |
|---|---|---|
| Normal | 32.5 ± 7.9 | - |
| Control | 395.4 ± 81.0 | - |
| Compound 1 (10 mg/kg) | 202.4 ± 93.1 | 53.2 ± 25.7 |
| Compound 1 (30 mg/kg) | 166.6 ± 43.6 | 63.0 ± 12.0 |

The test results indicate that the compound of the present invention is considered to be clinically applied to a variety of clinical respiratory diseases involving hyper-secretion of mucus from the respiratory tract by virtue of inhibitory effect on hyper-secretion of respiratory tract mucus.

| Drug Preparation Ex. 1 (Tablets) | |
|---|---|
| Compound 1 | 200 mg |
| Corn starch | 50 mg |
| Microcrystalline cellulose | 50 mg |
| Hydroxypropyl cellulose | 15 mg |
| Lactose | 47 mg |
| Talc | 2 mg |
| Magnesium stearate | 2 mg |
| Ethyl cellulose | 30 mg |
| Unsaturated glyceride | 2 mg |
| Titanium dioxide | 2 mg |

Tablets (400 mg/tablet) of the above formulation were prepared through a routine method.

| Drug Preparation Ex. 2 (Granules) | |
|---|---|
| Compound 2 | 300 mg |
| Lactose | 540 mg |
| Corn starch | 100 mg |
| Hydroxypropyl cellulose | 50 mg |
| Talc | 10 mg |

Granules (1,000 mg/sachet) of the above formulation were prepared through a routine method.

| Drug Preparation Ex. 3 (Capsules) | |
|---|---|
| Compound 3 | 200 mg |
| Lactose | 30 mg |
| Corn starch | 50 mg |
| Microcrystalline cellulose | 10 mg |
| Magnesium stearate | 3 mg |

Capsules (293 mg/capsule) of the above formulation were prepared through a routine method.

| Drug Preparation Ex. 4 (Injections) | |
|---|---|
| Compound 4 | 100 mg |
| Sodium chloride | 3.5 mg |
| Distilled water for injection quant. suff. | |

Injections (2 mL/ampule) of the above formulation were prepared through a routine method.

| Drug Preparation Ex. 5 (Syrups) | |
|---|---|
| Compound 5 | 200 mg |
| Refined sugar | 60 g |
| Ethyl p-hydroxybenzoate | 5 mg |
| Butyl p-hydroxybenzoate | 5 mg |
| Perfume | quant. suff. |
| Colorant | quant. suff. |
| Purified water | quant. suff. |

Syrups of the above formulation were prepared through a routine method.

| Drug Preparation Ex. 6 (Suppositories) | |
|---|---|
| Compound 6 | 300 mg |
| Witepsol W-35 | 1,400 mg |

(Registered trademark, a mixture of mono-, di-, and triglyceride of saturated fatty acid (lauric acid to staeric acid), product of Dynamit Nobel A.G.)

Suppositories of the above formulation were prepared through a routine method.

| Drug Preparation Ex. 7 (Aerosols) | |
|---|---|
| Compound 1 | 100 mg |
| Miglyol 812 | 200 mg |
| HFA-227 | quant. suff. |
| (20 mL/aerosol) | |

Compound 1 was crushed to form a micropowder of 5 µm or less through a conventional method, and the micropowder was kneaded with Miglyol 812. Subsequently, the kneaded product was charged into a spraying apparatus, and HFA-227 cooled in advance to -20°C was charged to the apparatus. The apparatus was closed with a valve, to thereby produce an aerosol preparation of the above formulation.

| Drug Preparation Ex. 8 (Liquid inhalation preparations) | |
|---|---|
| Compound 2 | 100 mg |
| Benzalkonium chloride | 10 mg |
| Purified water | quant. suff. |
| (100 mL/inhalation preparation) | |

Purified water was added to Compound 2 and benzalkonium chloride, and the mixture was stirred for dissolution, thereby producing a liquid inhalation preparation of the above formulation.

| Drug Preparation Ex. 9 (Powder inhalation preparations) | |
|---|---|
| Compound 3 | 5 mg |
| Lactose | 95 mg |
| (100 mg/inhalation preparation) | |

Compound 3 was pulverized by means of a jet-mill (product of Fuji Sangyo) to form a micropowder of 5 µm or less, and the micropowder was mixed with lactose, thereby producing a powder inhalation preparation of the above formulation.

## Claims

1. A mucin production inhibitor comprising, as an active ingredient, a benzimidazole derivative represented by formula (1): 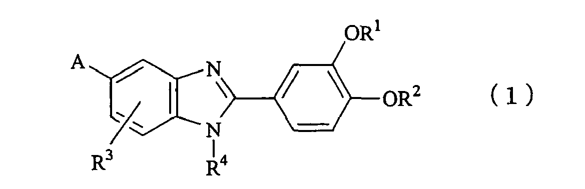 (wherein A represents a triazole group; R¹ and R² may be identical to or different from each other, and each represents an aliphatic hydrocarbon group which may have an alicyclic hydrocarbon group, or an alicyclic hydrocarbon group; R³ represents a hydrogen atom or a substituent; and R⁴ represents a hydrogen atom or a protective group for the nitrogen atom) or a pharmacologically acceptable salt thereof.

2. The mucin production inhibitor as described in claim 1, wherein A is 1,2,4-triazol-1-yl; R¹ and R² is identical to or different from each other, and each represents a linear or branched lower C1-C6 alkyl group optionally having a C3-C7 mono-ring alicyclic hydrocarbon group, or a C3-C7 mono-ring alicyclic hydrocarbon group; R³ is a hydrogen atom; and R⁴ is a hydrogen atom.

3. The mucin production inhibitor as described in claim 1, wherein A is 1,2,4-triazol-1-yl; R¹ and R² is identical to or different from each other, and each represents methyl, isobutyl, 3-pentyl, cyclopropylmethyl, or cyclopentyl; R³ is a hydrogen atom; and R⁴ is a hydrogen atom.

4. A mucin production inhibitor comprising, as an active ingredient, 2-(3-cyclopropylmethyloxy-4-methoxyphenyl)-5-(1,2,4-triazol-1-yl)benzimidazole or a pharmacologically acceptable salt thereof.

5. The mucin production inhibitor as described in any one of claims 1 to 4, which is an agent for preventing or treating a respiratory disease accompanying hyper-secretion of mucus from the respiratory tract epithelium.

6. The mucin production inhibitor as described in claim 5, wherein the respiratory disease accompanying hyper-secretion of mucus from the respiratory tract epithelium is chronic obstructive pulmonary disease, chronic respiratory tract infections, chronic bronchitis, chronic sinusitis, pneumonia, pulmonary tuberculosis, pneumoconiosis, and pulmonary fibrosis.

7. An agent for ameliorating a symptom in relation to a respiratory disease accompanying hyper-secretion of mucus from the respiratory tract epithelium, the symptom being stagnation of secreted respiratory tract mucus, difficulty in sputum expectoration, or accumulation of sputum, the agent comprising, as an active ingredient, a benzimidazole derivative represented by formula (1): 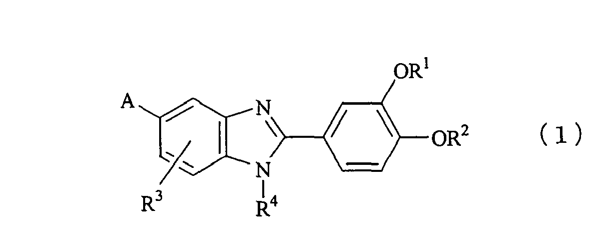 (wherein A represents a triazole group; R¹ and R² may be identical to or different from each other, and each represents an aliphatic hydrocarbon group which may have an alicyclic hydrocarbon group, or an alicyclic hydrocarbon group; R³ represents a hydrogen atom or a substituent; and R⁴ represents a hydrogen atom or a protective group for the nitrogen atom) or a pharmacologically acceptable salt thereof.

8. The agent for ameliorating a symptom as described in claim 7, wherein the respiratory disease accompanying hyper-secretion of mucus from the respiratory tract epithelium is chronic obstructive pulmonary disease, chronic respiratory tract infections, chronic bronchitis, chronic sinusitis, pneumonia, pulmonary tuberculosis, pneumoconiosis, and pulmonary fibrosis.

9. An expectorant comprising, as an active ingredient, a benzimidazole derivative represented by formula (1): 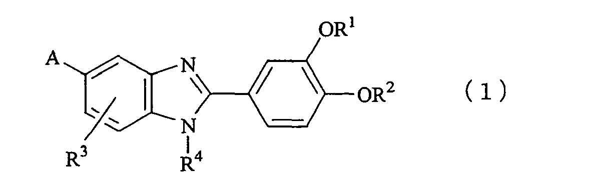 (wherein A represents a triazole group; R¹ and R² may be identical to or different from each other, and each represents an aliphatic hydrocarbon group which may have an alicyclic hydrocarbon group, or an alicyclic hydrocarbon group; R³ represents a hydrogen atom or a substituent; and R⁴ represents a hydrogen atom or a protective group for the nitrogen atom) or a pharmacologically acceptable salt thereof.

10. A method for treating a respiratory disease accompanying hyper-secretion of mucus from the respiratory tract epithelium, the method comprising administering a mucin production inhibitor as recited in any one of claims 1 to 4 in an effective amount.

11. A method for treating a respiratory disease accompanying hyper-secretion of mucus from the respiratory tract epithelium as described in claim 10, wherein the respiratory disease accompanying hyper-secretion of mucus from the respiratory tract epithelium is chronic obstructive pulmonary disease, chronic respiratory tract infections, chronic bronchitis, chronic sinusitis, pneumonia, pulmonary tuberculosis, pneumoconiosis, and pulmonary fibrosis.

12. A method for ameliorating a symptom in relation to a respiratory disease accompanying hyper-secretion of mucus from the respiratory tract epithelium, the symptom being stagnation of secreted respiratory tract mucus, difficulty in sputum expectoration, or accumulation of sputum, the method comprising administering, in an effective amount, a benzimidazole derivative represented by formula (1): 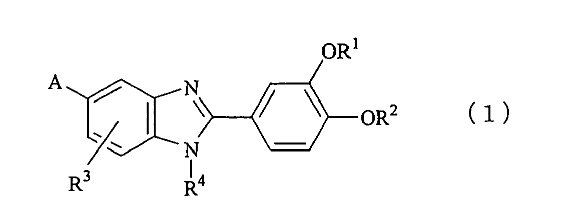 (wherein A represents a triazole group; R¹ and R² may be identical to or different from each other, and each represents an aliphatic hydrocarbon group which may have an alicyclic hydrocarbon group, or an alicyclic hydrocarbon group; R³ represents a hydrogen atom or a substituent; and R⁴ represents a hydrogen atom or a protective group for the nitrogen atom) or a pharmacologically acceptable salt thereof.

13. The method for ameliorating a symptom as described in claim 12, wherein the respiratory disease accompanying hyper-secretion of mucus from the respiratory tract epithelium is chronic obstructive pulmonary disease, chronic respiratory tract infections, chronic bronchitis, chronic sinusitis, pneumonia, pulmonary tuberculosis, pneumoconiosis, and pulmonary fibrosis.

14. Use of a benzimidazole derivative represented by formula (1): 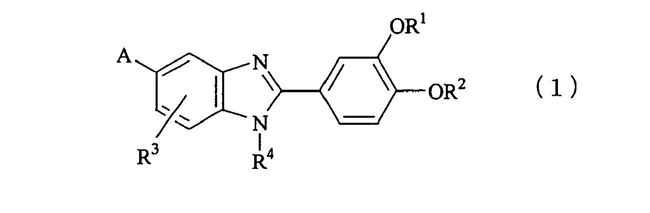 (wherein A represents a triazole group; R¹ and R² may be identical to or different from each other, and each represents an aliphatic hydrocarbon group which may have an alicyclic hydrocarbon group, or an alicyclic hydrocarbon group; R³ represents a hydrogen atom or a substituent; and R⁴ represents a hydrogen atom or a protective group for the nitrogen atom) or a pharmacologically acceptable salt thereof for the manufacture of a mucin production inhibitor.

15. Use of a benzimidazole derivative represented by formula (1): 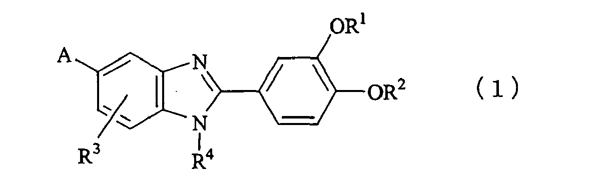 (wherein A represents a triazole group; R¹ and R² may be identical to or different from each other, and each represents an aliphatic hydrocarbon group which may have an alicyclic hydrocarbon group, or an alicyclic hydrocarbon group; R³ represents a hydrogen atom or a substituent; and R⁴ represents a hydrogen atom or a protective group for the nitrogen atom) or a pharmacologically acceptable salt thereof for the manufacture of a mucin production inhibitor for ameliorating a symptom in relation to a respiratory disease accompanying hyper-secretion of mucus from the respiratory tract epithelium, the symptom being stagnation of secreted respiratory tract mucus, difficulty in sputum expectoration, or accumulation of sputum.

16. The use of claim 15, wherein the respiratory disease accompanying hyper-secretion of mucus from the respiratory tract epithelium is chronic obstructive pulmonary disease, chronic respiratory tract infections, chronic bronchitis, chronic sinusitis, pneumonia, pulmonary tuberculosis, pneumoconiosis, and pulmonary fibrosis.
